# EUROPÄISCHE PATENTSCHRIFT

(11) **EP 0 362 835 B1**
(45) Veröffentlichungstag und Bekanntmachung des Hinweises auf die Patenterteilung: **15.06.1994**
(21) Anmeldenummer: 89118446.7
(22) Anmeldetag: 04.10.1989
(51) Int. Cl.: B65D 47/34, A61B 19/00, B67D 5/02, A47K 5/12

(54) **Behältnis und Vorratsbehältnis für Desinfektionsmittel, Seifencreme, Handcreme, Hautpflegemittel und dergleichen**
Container and productcontainer for a disinfectant product, soap cream, hand cream, skin care product or the like
Conteneur et conteneur pour produits désinfectants, crème savon, crème de main, produits pour soigner la peau, ou similaires

(30) Priorität: 04.10.1988 DE 3833663
(43) Veröffentlichungstag der Anmeldung: 11.04.1990
(73) Patentinhaber: DESOMED AG, CH-4106 Therwil (CH)
(72) Erfinder: Ensslin, Konrad, D-7062 Rudersberg (DE)
(74) Vertreter: Jackisch-Kohl, Anna-Katharina

(56) Entgegenhaltungen:
- FR-A- 1 287 487
- FR-A- 1 540 627
- FR-A- 2 417 443
- GB-A- 2 080 249
- GB-A- 2 155 117
- US-A- 4 588 358

## Beschreibung

Die Erfindung betrifft ein Behältnis und ein flexibles, beutelartiges Vorratsbehältnis für Mittel nach dem Oberbegriff des Anspruches 1.

Dieses bekannte Behältnis (FR-A-1 287 487) besteht aus zwei gelenkig miteinander verbundenen Teilen, die das flexible Vorratsbehältnis aufnehmen. Der Hals des Behältnisses ist als Gewindeabschnitt geformt, auf den das Schraubteil geschraubt wird, mit dem die beiden Behälterteile zusammengehalten werden. Das Vorratsbehältnis ist mit einem Hals versehen, der mit einem Außengewinde versehen ist, das in das Gewinde des Behältnishalses eingreift. Auf diese Weise wird auch das im Behältnis befindliche Vorratsbehältnis durch das Schraubteil gegen Hineinrutschen in das Behältnis gesichert. Solange das Schraubteil nicht aufgeschraubt ist, können die beiden Behälterteile leicht gegeneinander verschwenkt werden. Ist das Vorratsbehältnis in das starre Behältnis eingesetzt, besteht darum die Gefahr, daß der eine Behälterteil unbeabsichtigt wegschwenkt und das Vorratsbehältnis herausfällt. Der Hals des Vorratsbehältnisses ist als starres Teil ausgebildet, wodurch sich erhebliche Nachteile hinsichtlich der Beseitigung des leeren Vorratsbehältnisses ergeben. Aufgrund dieses formsteifen Halses läßt sich das leere Vorratsbehältnis nicht klein zusammenlegen. Darum ergibt sich bei entsprechend großen Abfallmengen ein entsprechend großes Abfallvolumen.

Der Erfindung leigt die Aufgabe zugrunde, das gattungsgemäße Behältnis und Vorratsbehältnis so auszubilden, daß im Behältnis einerseits das flexible Vorratsbehältnis sicher für die Handhabung untergebracht und gehalten wird, andererseits das leere Vorratsbehältnis optimal bei kleinstem Abfallvolumen entsorgt werden kann.

Diese Aufgabe wird beim gattungsgemaßen Behältnis und Vorratsbehältnis erfindungsgemäß mit den kennzeichnenden Merkmalen des Anspruches 1 gelöst.

Beim erfindungsgemäßen Behältnis werden die Behältnisteile durch die Rasteinrichtungen in der Schließlage unmittelbar aneinander gehalten. Wird das volle Vorratsbehältnis in das formsteife Behältnis eingesetzt, dann wird das Vorratsbehältnis infolge der rastenden Verbindung der Behältnisteile zuverlässig im Behältnis gehalten, auch wenn das Schraubteil noch nicht auf den Hals des Behältnisses geschraubt ist. Dadurch besteht nicht die Gefahr, daß sich das erfindungsgemäße Behältnis beim Einsetzen des Vorratsbehältnisses unbeabsichtigt öffnet. Zur Sicherung des flexiblen Vorratsbehältnisses im Behältnis dient die Ringwulst am Hals des Vorratsbehältnisses. Sie beeinträchtigt das Abfallvolumen des Vorratsbehältnisses nicht, da sie ebenso wie das gesamte Vorratsbehältnis äußerst flexibel ist. Darum lassen sich die leeren Vorratsbehältnisse sehr klein zusammenlegen, so daß sie auf dem Müll nur ein äußerst geringes Volumen beanspruchen. Der Hals des Behältnisses dient als Auflager für die Ringwulst des Vorratsbehältnisses, das dadurch sehr einfach am Zurückrutschen in das Behältnis gehindert wird.

Die Erfindung wird anhand zweier in den Zeichnungen dargestellter Ausführungsbeispiele näher erläutert. Es zeigen
- Fig. 1: in Explosionsdarstellung ein erfindungsgemäßes Behältnis zur Aufnahme eines flexiblen Vorratsbehältnisses,
- Fig. 2: das Vorratsbehältnis, das in einen Teil des Behältnisses gemäß Fig. 1 eingesetzt ist,
- Fig. 3: in vergrößerter und vereinfachter Darstellung den oberen Teil des Behältnisses mit eingesetztem Vorratsbehältnis,
- Fig. 4: in perspektivischer und explosiver Darstellung eine zweite Ausführungsform eines erfindungsgemäßen Behältnisses.

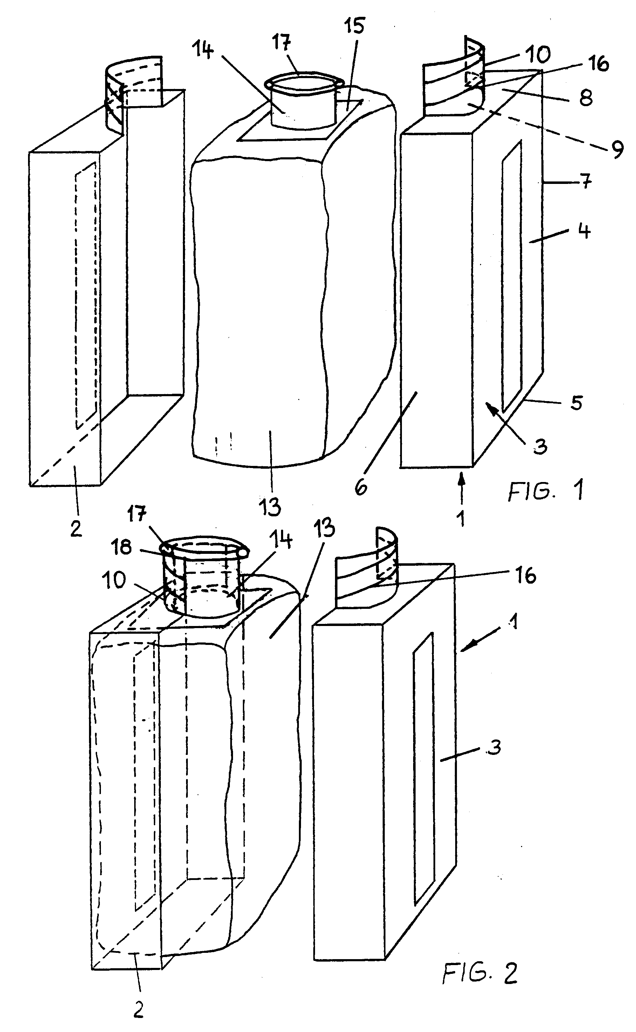

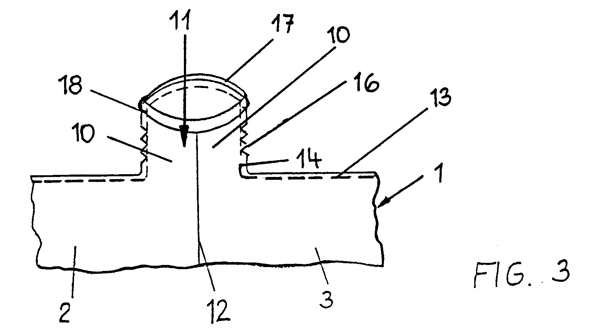

Das Behältnis 1 gemäß den Fig. 1 bis 3 besteht aus zwei Hälften 2 und 3. Sie sind gleich ausgebildet und spiegelsymmetrisch zueinander angeordnet. Darum wird im folgenden nur die eine Hälfte 3 näher erläutert. Sie hat eine Rückwand 4, einen Boden 5, Seitenwände 6, 7 und eine Deckwand 8. In der Deckwand 8 ist eine halbkreisförmige Ausnehmung 9 vorgesehen, an deren Rand ein teilzylindrischer Ansatz 10 anschließt. Die beiden Hälften 2 und 3 werden so aneinandergesetzt, daß sie mit ihren Seitenwänden 6, 7, ihrem Boden 5 und der Deckwand 8 aneinanderstoßen und auf diese Weise das Behältnis 1 bilden, das allseitig geschlossen ist. Die beiden Ansätze 10 bilden im zusammengefügten Zustand einen Hals 11 (Fig. 3). Die Stoßstelle zwischen den beiden Hälften 2, 3 ist in Fig. 3 mit 12 bezeichnet. Vorzugsweise sind die beiden Hälften 2, 3 an ihren aneinanderstoßenden Rändern überlappend ausgebildet, so daß eine Art Dichtung gebildet wird, die bei einem versehentlichen Aufreißen eines in das Behältnis 1 einzusetzenden Vorratsbehältnisses 13 den Austritt des Mittels aus dem Behältnis 1 verhindern. In die Stoßkanten können auch eine oder mehrere Dichtungen eingelegt sein, um das Behältnis 1 trotz seiner zweiteiligen Ausbildung absolut dicht zu halten.

Das Vorratsbehältnis 13 ist flexibel ausgebildet und besteht aus dünnem, reißfestem Kunststoff, zum Beispiel Thermoplast, wie Polyäthylen. Das flexible Vorratsbehältnis 13 ist nach Art eines Beutels ausgebildet und trägt einen Hals 14. Er sitzt auf einer verstärkten Kunststoffplatte 15, die zwar etwas steifer als das Vorratsbehältnis 13 selbst ist, jedoch immer noch flexibel ausgebildet ist.

Fig. 2 zeigt eine Möglichkeit, das Vorratsbehältnis 13 möglichst einfach im Behältnis 1 unterzubringen. Zweckmäßig wird hierzu das Vorratsbehältnis 13 zunächst in die eine Hälfte 2 des Behältnisses 1 so eingesetzt, daß der Hals 14 im teilzylindrischen Ansatz 10 der Behältnishälfte 2 liegt. Da das Vorratsbehältnis 13 hierbei schon teilweise von den Seitenwänden 6, 7, der Deckwand 8, der Rückwand 4 und dem Boden 5 der Hälfte 2 umgeben ist, läßt sich anschließend die andere Behältnishälfte 3 leicht aufsetzen, ohne das Vorratsbehältnis 13 zu beschädigen. Die beiden Behältnishälften 2, 3 sind mit (nicht dargestellten) Rasteinrichtungen versehen, mit denen die beiden Hälften 2, 3 zuverlässig zusammengehalten werden.

Das Behältnis 1 mit dem eingesetzten flexiblen beutelförmigen Vorratsbehältnis 13 kann beispielsweise im Haushalt eingesetzt werden. Das Vorratsbehältnis 13 kann mit einem Dispenser, einem Spritzverschluß, einer Sprühpistole, einem Dosierer, einem Spraykopf oder dergleichen ausgerüstet werden. Im Vorratsbehältnis 13 können Hand- und Körperreinigungsmittel, Haut- und Pflegemittel, Desinfektionsmittel jeder Art für Hände, Flächen, Instrumente oder dergleichen, Duftstoffe, Kosmetikpräparate und dergleichen vorgesehen sein.

Das Behältnis 1 wird als separater Behälter verwendet, der jede beliebige Umrißform haben kann.

Ist das Vorratsbehältnis 13 leer, dann wird das Behältnis 1 geöffnet und das leere Vorratsbehältnis gegen ein volles Vorratsbehältnis 13 ausgewechselt. Da das Vorratsbehältnis 13 aus folienartigem, flexiblem Material besteht, nimmt das Vorratsbehältnis nur wenig Platz in Anspruch. Es können darum Hunderte solcher Vorratsbehältnisse zu einem kleinen Paket gebündelt werden, das somit nur geringsten Platzbedarf hat. Eine vergleichbare Menge an herkömmlichen Vorratsflaschen würde demgegenüber einen um Größenordnungen größeren Raumbedarf haben.

Der Hals 11 des Behältnisses 1 ist mit einem Außengewinde 16 versehen. Der Hals 14 des Vorratsbehältnisses 13 besteht aus dem gleichen folienartigen, flexiblen Material wie das Vorratsbehältnis selbst. Um ein Durchrutschen des Halses 14 des Vorratsbehältnisses 13 durch den Hals 11 des Behältnisses 1 zu verhindern, ist der Hals 14 am freien Ende mit einer umlaufenden, wulstartigen Verdickung 17 versehen, die auf der Stirnseite 18 des Halses 11 des Behältnisses 1 aufliegt. Die Verdickung 17 ist so steif, daß sie nicht durch den Hals 11 in das Behältnis 1 rutschen kann. Vorzugsweise kann die Verdikkung 17 als Dichtung dienen, wenn auf den Hals 11 des Behältnisses 1 beispielsweise ein Spraykopf geschraubt wird.

Die von der Verdickung 17 umgebene Öffnung des Vorratsbehältnisses 13 ist zunächst mit einem folienartigen Deckel verschlossen, der nach dem Einsetzen des Vorratsbehältnisses 13 in das Behältnis 1 abgezogen werden kann.

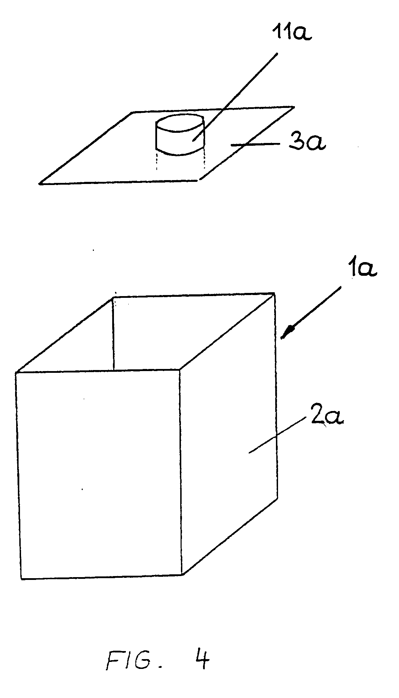

Bei der Ausführungsform gemäß Fig. 4 ist der Behältnisteil 2a als nahezu geschlossener Behälter ausgebildet. Der andere Behältnisteil 3a kann vollständig vom Behältnisteil 2a abgenommen werden. In das dadurch nach oben offene Behältnis 1a läßt sich das Vorratsbehältnis einfach einsetzen. Anschließend wird der einen Deckel bildende Behältnisteil 3a auf den Behältnisteil 2a aufgesetzt. Der Behältnisteil 3a weist den Hals 11a auf, der mit Außengewinde versehen ist. Das Vorratsbehältnis wird dann in gleicher Weise im Behältnis 1a gehalten, wie dies beim vorigen Ausführungsbeispiel erläutert worden ist.

Die Behältnisteile können auch gelenkig miteinander verbunden sein. Sind die Behältnisse, wie in den Fig. 1 bis 4 dargestellt, aus zwei voneinander getrennten Teilen gebildet, dann kann der eine Behältnisteil beispielsweise an einer Wand befestigt sein, so daß nach dem Einsetzen des jeweiligen Vorratsbehältnisses der andere Behältnisteil am vormontierten Behältnisteil angesetzt werden kann. Es ist ferner möglich, an einer Wand oder dergleichen eine Haltevorrichtung vorzusehen, in welche die beschriebenen Behältnisse eingehängt werden können. Eine solche Variante hat den Vorteil, daß das Behältnis abgenommen und einfach gereinigt werden kann.

Es besteht die Möglichkeit, das Behältnis, das beispielsweise mit einem Dispenser ausgerüstet ist, zusammen mit dem darin befindlichen Vorratsbehältnis beispielsweise an einer Seitenwand eines Putzeimers lösbar zu befestigen. Dann kann beispielsweise ein Desinfektionsmittel direkt in die im Putzeimer befindliche Flüssigkeit eingegeben werden. Ein solcher Putzeimer mit eingehängtem Behältnis könnte auch gleichzeitig als Versandbehälter dienen.

Den anhand der Fig. 1 bis 4 beschriebenen Ausführungsformen ist gemeinsam, daß flexible Vorratsbehältnisse verwendet werden können, die keine Abfallprobleme mit sich bringen. Die leeren Vorratsbehältnisse können sehr klein zusammengefaltet bzw. gebündelt werden, so daß auch bei einer großen Zahl von Vorratsbehältnissen nur wenig Raum für diesen Abfall benötigt wird. Dies ist insbesondere für Krankenhäuser, in denen regelmäßig große Mengen von Vorratsbehältnissen verbraucht werden, ein nicht zu unterschätzender Vorteil.

## Patentansprüche

1. Behältnis (1, 1a) und flexibles, beutelartiges Vorratsbehältnis (13) für Mittel, wobei das Behältnis (1, 1a) aus mindestens zwei Teilen (2, 3; 2a, 3a) zusammengesetzt ist, von denen zumindest ein Teil (2, 3; 2a, 3a) zum Einsetzen des Vorratsbehältnisses (13) in eine eine Einsetzöffnung des Behältnisses (1, 1a) freigebende Lage bewegbar ist, das mit einem ein Außengewinde (16) aufweisenden Hals (11, 11a) versehen ist, der einen mit einer Verdickung (17) versehenen Hals (14) des Vorratsbehältnisses (13) umschließt und auf den ein Schraubteil schraubbar ist,
dadurch gekennzeichnet, daß die beiden Teile (2, 3; 2a, 3a) des Behältnisses (1, 1a) durch Rasteinrichtungen in der Schließlage unmittelbar aneinander gehalten sind, und daß die Verdickung des Halses (14) des Vorratsbehältnisses (13) eine Ringwulst (17) ist, die auf der Stirnseite (18) des Halses (11, 11a) des Behältnisses (1, 1a) aufliegt.

2. Behältnis und flexibles, beutelartiges Vorratsbehältnis nach Anspruch 1,
dadurch gekennzeichnet, daß die beiden Behältnisteile (2, 3; 2a, 3a) dichtend miteinander zu verbinden sind.

3. Behältnis und flexibles, beutelartiges Vorratsbehältnis nach Anspruch 1 oder 2,
dadurch gekennzeichnet, daß der Hals (14) des Vorratsbehältnisses (13) flexibel ausgebildet ist.

4. Behältnis und flexibles, beutelartiges Vorratsbehältnis nach einem der Ansprüche 1 bis 3,
dadurch gekennzeichnet, daß der eine Teil (3a) eine Wand des Behältnisses (1a) bildet.

## Claims

1. Container (1, 1a) and flexible, bag-like storage container (13) for products, the container (1, 1a) being composed of at least two parts (2, 3; 2a, 3a), whereof at least one part (2, 3; 2a, 3a) for inserting the storage container (13) can be moved into a position exposing an insertion opening of the container (1, 1a), which is provided with a neck (11, 11a) comprising an external thread (16), which neck (11, 11a) surrounds a neck (14) of the storage container (13) provided with a thickened portion (17) and onto which neck (14) a screw part can be screwed, characterised in that the two parts (2, 3; 2a, 3a) of the container (1, 1a) are held directly one against the other in the closed position by locking devices, and that the thickened portion of the neck (14) of the storage container (13) is an annular bead (17), which rests on the end face (18) of the neck (11, 11a) of the container (1, 1a).

2. Container and flexible, bag-like storage container according to Claim 1, characterised in that the two container parts (2, 3; 2a, 3a) have to be connected to each other in a sealed manner.

3. Container and flexible, bag-like storage container according to Claim 1 or 2, characterised in that the neck (14) of the storage container (13) has a flexible construction.

4. Container and flexible, bag-like storage container according to one of Claims 1 to 3, characterised in that the one part (3a) forms one wall of the container (1a).

## Revendications

1. Conteneur (1, 1a) et réservoir (13) souple en forme de sachet destiné à recevoir des produits, le conteneur (1, 1a) étant assemblé à partir d'au moins deux éléments (2, 3; 2a, 3a) dont au moins l'un (2, 3; 2a, 3a) peut être amené dans une position qui libère l'ouverture du conteneur (1, 1a) pour l'insertion du réservoir (13) et est muni d'un col (11, 11a) pourvu d'un filetage extérieur (16), qui entoure un col (14) du réservoir (13) avec un renflement (17) et peut être vissé sur un élément à vis, **caractérisés en ce** que, dans la position de fermeture, les deux éléments (2, 3; 2a, 3a) du conteneur (1, 1a) sont directement maintenus ensemble par des dispositifs à crans, et que le renflement du col (14) du réservoir (13) est conformé en bourrelet annulaire (17) qui repose sur la face frontale (18) du col (11, 11a) du conteneur (1, 1a).

2. Conteneur et réservoir souple en forme de sachet selon la revendication 1, caractérisés en ce que les deux éléments (2, 3; 2a, 3a) du conteneur peuvent être raccordés de manière étanche.

3. Conteneur et réservoir souple en forme de sachet selon l'une des revendications 1 ou 2, caractérisés en ce que le col (14) du réservoir (13) est souple.

4. Conteneur et réservoir souple en forme de sachet selon l'une des revendications 1 à 3, caractérisés en ce que l'un des éléments (3a) constitue une paroi du conteneur (1a).
